# EUROPEAN PATENT APPLICATION

(11) **EP 4 266 318 A1**
(43) Date of publication of application: **25.10.2023**
(21) Application number: 22169567.9
(22) Date of filing: 22.04.2022
(51) Int. Cl.: G16H 10/20

(54) **METHOD AND SYSTEM FOR DETERMINING PATIENT SELECTION CRITERIA FOR A CLINICAL TRIAL**

(71) Applicant: Sensyne Health Group Limited, Oxford OX4 4GE (GB)
(72) Inventor: JAVER, Avelino, Oxford, OX4 4GE (GB); CARR, Oliver, Oxford, OX4 4GE (GB); FARRANT, James, Oxford, OX4 4GE (GB); PARSONS, Owen, Oxford, OX4 4GE (GB); HOSKINS, Coran, Oxford, OX4 4GE (GB); BAXTER, Janie, Oxford, OX4 4GE (GB); DÜRICHEN, Robert, Oxford, OX4 4GE (GB); BARLOW, Nathan, Oxford, OX4 4GE (GB)
(74) Representative: Lewis, Stefanie Janneke

(57) **Abstract**

A method for determining patient selection criteria for a clinical trial having at least one trial outcome is described. The method comprises obtaining a set of patient selection criteria comprising at least one primary criterion, and accessing a dataset comprising a plurality of data items for each patient, the plurality of data items comprising first data item(s) corresponding to the trial outcome(s) and second data item(s) corresponding to the patient selection criteria. The method comprises identifying a set of patients of the plurality of patients in the dataset based on the at least one second data item, performing a clustering analysis on the set of patients to identify a cluster of patients, determining whether the identified cluster of patients is clinically meaningful, and upon determining that the identified cluster of patients is clinically meaningful, determining at least one additional patient selection criterion based on the identified cluster of patients and modifying the set of patient selection criteria by adding the at least one additional patient selection criterion to the set of patient selection criteria.

## Description

### Technical Field

This disclosure relates generally to determining patient selection criteria for a clinical trial, and specifically to a computer-implemented method and system for determining patient selection criteria for a clinical trial. The determined patient selection criteria may be used for selecting patients to enroll in a clinical trial, for example.

### Background

The development and approval of new drugs is a crucial aspect of medical advancement. It is essential to ensure that any new drugs are thoroughly tested to ensure their efficacy, safety, and to identify any side effects. As such, the design and running of clinical trials is a long, expensive aspect of drug development and drug approval. Up to 50% of research and development expenditure in the development and production of a new drug is typically used for clinical trials. A substantial proportion of this cost relates to identifying and recruiting patients for the clinical trial and performing the trial. Moreover, there is a high risk of failure at this stage of drug development - over one-third of phase III compounds (e.g. drugs being tested in clinical trials) fail.

Patients are typically recruited for a clinical trial by a specific set of inclusion and exclusion criteria, referred to as patient selection criteria. The patient selection criteria are generally defined based on expert knowledge and the results of previous studies, such as clinical studies or laboratory studies.

However, selecting patients based on expert knowledge and the results of previous studies can often be vague, ambiguous, overly restrictive and/or complex. The inclusion and exclusion of particular criteria is often subjective. In particular, the patient selection criteria for a particular clinical trial is often a copy of the patient selection criteria used in a previous study without substantial adaptation. This approach can lead to unintended biasing, for example by reinforcing a converging selection of certain population subgroups.

There is therefore a need to provide an improved method for determining patient selection criteria which addresses some or all of the shortcomings listed above.

### Summary

Aspects and optional features of the present disclosure are described in the accompanying claims.

One aspect of the present disclosure provides a computer-implemented method for determining patient selection criteria for a clinical trial, the clinical trial having at least one trial outcome. The method comprises obtaining a set of patient selection criteria. The set of patient selection criteria comprises at least one primary criterion. The method further comprises accessing a dataset comprising a plurality of data items for each of a plurality of patients. The plurality of data items comprises at least one first data item corresponding to the at least one trial outcome and at least one second data item corresponding to the set of patient selection criteria. The method further comprises identifying a set of patients of the plurality of patients in the dataset based on the at least one second data item, performing a clustering analysis on the set of patients to identify a cluster of patients and determining whether the identified cluster of patients is clinically meaningful. Upon determining that the identified cluster of patients is clinically meaningful, the method further comprises determining at least one additional patient selection criterion based on the identified cluster of patients, and modifying the set of patient selection criteria by adding the at least one additional patient selection criterion to the set of patient selection criteria.

Optionally, the method may further comprise outputting the modified set of patient selection criteria to a user.

Optionally, the method may further comprise selecting patients according to the modified set of patient selection criteria. For example, patients may be selected from a database, such as a local or global health database, a government database, a database of a health care provider or the like.

Optionally, performing the cluster analysis may comprise using at least one of a machine learning cluster analysis. For example, the machine learning cluster analysis may be a simple k-means cluster analysis.

In some implementations, determining at least one additional patient selection criterion may further comprise training a surrogate model using the plurality of data items, generating a set of inclusion and/or exclusion criteria based on the trained surrogate model, and modifying the set of inclusion and/or exclusion criteria by generating and outputting an interactive user interface enabling features to be added and/or subtracted from the set of inclusion and/or exclusion criteria, adaptively displaying an indication of clinical meaningfulness of the set of inclusion and/or exclusion criteria, and obtaining user input via the interactive user interface, and providing the modified set of inclusion and/or exclusion criteria as the at least one additional patient selection criterion.

Optionally, determining whether the identified cluster of patients is clinically meaningful may comprise calculating a statistical significance score and comparing the calculated statistical significance score to a threshold statistical significance.

Optionally, determining whether the identified cluster of patients is clinically meaningful may comprise performing a feature enrichment analysis and/or a time-to-event analysis.

Optionally, determining whether the identified cluster of patients is clinically meaningful may comprise determining if one or more features are over- or under-represented in the cluster compared to the set of patients, the one or more features being associated with one or more data items of the plurality of data items.

Optionally, the set of patient selection criteria may further comprise at least one secondary criterion, the at least one secondary criterion associated with a further at least one data item of the plurality of data items. Identifying the set of patients of the plurality of patients in the dataset may comprise removing one or more of the at least one secondary criterion from the set of patient selection criteria, and identifying the set of patients of the plurality of patients in the dataset based on the second data item and the further at least one data items associated with any remaining secondary criteria. Modifying the set of patient criteria may further comprise removing the one or more of the at least one secondary criterion from the set of patient selection criteria.

Optionally, the method may further comprise obtaining a first mapping between the set of patient selection criteria and the plurality of data items and identifying the at least one second data item corresponding to the set of patient selection criteria according to the obtained first mapping, and/or obtaining a second mapping correlating the at least one trial outcome and at least one data item of the plurality of data items and identifying the at least one first data item according to the obtained second mapping.

Optionally, the method may further comprise identifying at least one quantitative indicator for the at least one trial outcome and identifying at least one first data item of the plurality of data items based on the identified at least one quantitative indicator.

Optionally, performing the cluster analysis may comprise selecting a cluster analysis algorithm based on at least one data type of the at least one first data item and/or the at least one second data item.

In another aspect of the present disclosure, one or more transitory or non-transitory computer-readable media are provided. The computer-readable media comprise instructions which, when executed by a computer, cause the computer to carry out any of the methods disclosed herein. Other aspects provide a computer program product comprising instructions which, when executed by a computer, cause the computer to carry out any of the methods disclosed herein.

In another aspect of the present disclosure, a device is provided which comprises an input/output subsystem configured to transmit and receive data, one or more processors, and a memory comprising instructions which, when executed, cause the one or more processors to execute any of the methods disclosed herein. Further aspects provide a system having a memory, one or more computer readable media as described above and a processor configured to implement the instructions.

### Brief description of the drawings

Some specific implementations are now described, by way of example only, with reference to the accompanying drawings, in which:
Figure 1 illustrates a flowchart of a method of determining patient selection criteria for a clinical trial according to embodiments of the disclosure;
Figure 2 illustrates a flowchart of a method of determining patient selection criteria for a clinical trial according to embodiments of the disclosure;
Figure 3 illustrates an example dataset according to embodiments of the disclosure;
Figure 4 illustrates a flowchart of further operations of a method of determining patient selection criteria for a clinical trial according to embodiments of the disclosure;
Figure 5 illustrates an example cluster visualization according to embodiments of the disclosure;
Figures 6a and 6b illustrate example plotted analyses of patient clusters according to embodiments of the disclosure;
Figure 7 illustrates a block diagram of a computing device; and
Figure 8 illustrates a diagram of a computer-readable medium.

### Detailed description

Aspects and features of the disclosure will be described below. In overview, and without limitation, the present application relates to a method for determining patient selection criteria for a clinical trial, the clinical trial having at least one outcome. The method comprises obtaining a set of patient selection criteria comprising at least one primary criterion. That is, an initial set of patient selection criteria obtained. The method further comprises accessing a dataset comprising a plurality of data items for a plurality of patients. The plurality of data items includes at least one first data item corresponding to the trial outcome(s) of the clinical trial, and at least one second data item corresponding to the patient selection criteria. The method further comprises identifying a set of patients of the plurality of patients based on the at least one second data item. That is, patients are identified according to the patient selection criteria. The method further comprises performing a clustering analysis on the set of patients to identify a cluster of patients. In some cases, multiple clusters may be identified. It is then determined whether the identified cluster(s) is clinically meaningful, and if so, at least one additional patient selection criterion is determined based on the identified cluster and the set of patient selection criteria is modified to include the at least one additional patient selection criterion. In other words, a clustering analysis may be used to identify patient selection criteria.

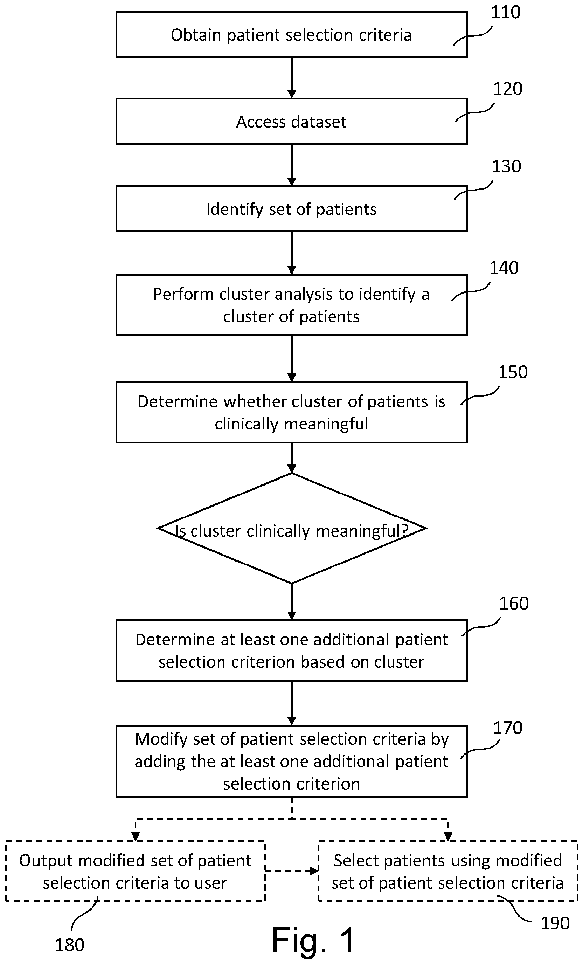

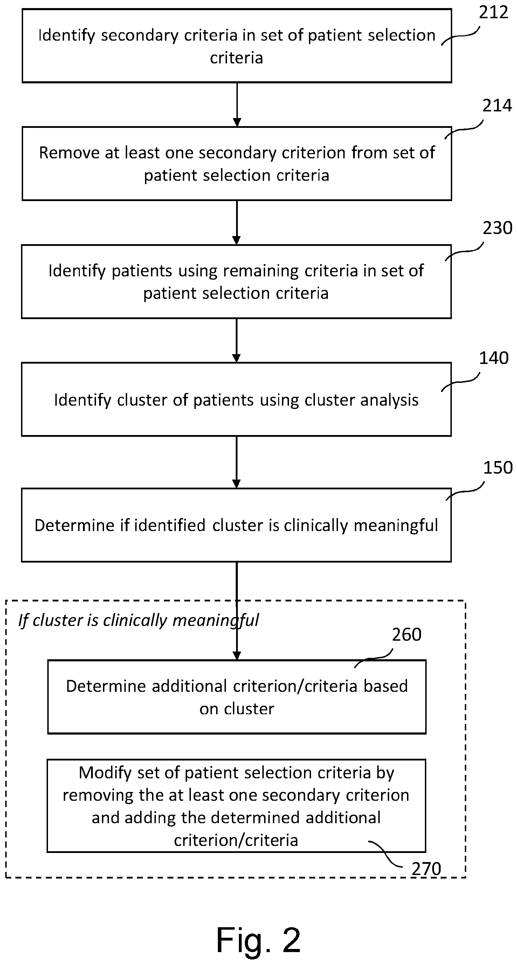

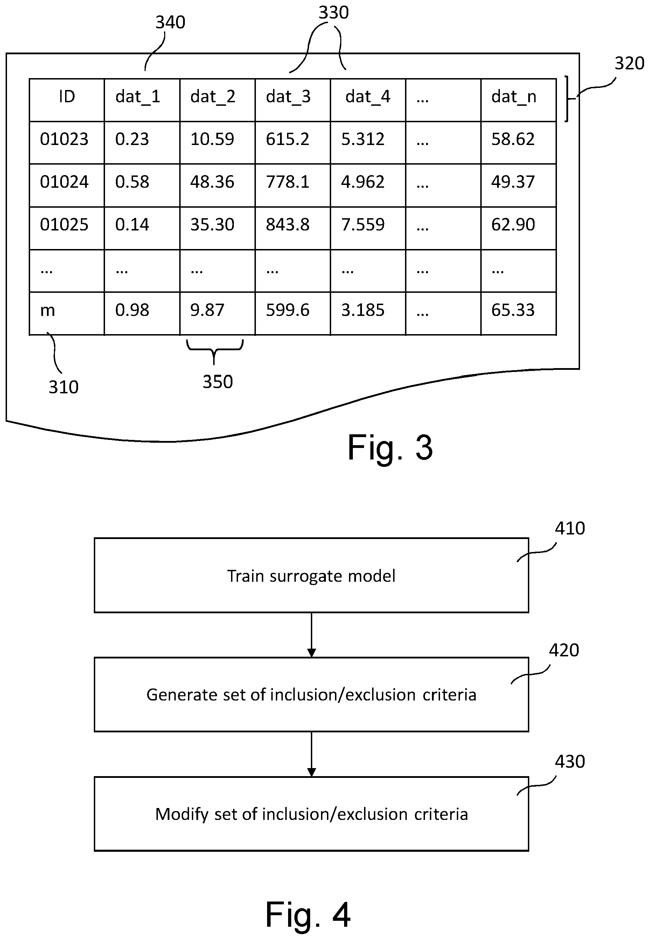

The description of Figure 1 will describe the general method, whilst the descriptions of Figures 2 and 4 will describe additional, optional, aspects of the general method. It is to be understood that elements of one or both of these additional aspects of the general method may be applicable in any implementation of the general method.

**Figure 1** illustrates a flowchart of a method 100 of determining patient selection criteria for a clinical trial according to embodiments of the disclosure.

A clinical trial has at least one trial outcome, which can be used to assess the success of the clinical trial. For example, a clinical trial may aim to prevent future stroke events, or to reduce mortality rates. Prevention of future stroke events or reduction of mortality are examples of trial outcomes.

The method 100 comprises, in operation 110, obtaining a set of patient selection criteria. The set of patient selection criteria may be obtained as a user input, e.g. by requesting a user to input one or more initial criteria, and/or from a database or from one or more previous studies. The patient selection criteria may be based on the purpose of the clinical trial. For example, for a clinical trial used to assess the efficacy of a new medication for diabetes, the patient selection criteria may include the criterion of a diagnosis of diabetes, for example type II diabetes. Other patient selection criteria may include, for example, an age range, a weight range or limit, a blood pressure range, or the like. The set of patient selection criteria comprises at least one primary criterion. As will be discussed with reference to Figure 2 below, the set of patient selection criteria may further comprise one or more secondary criteria. Throughout this description, a patient selection criterion (e.g. a primary criterion, a secondary criterion, an additional criterion, etc.) may refer to an inclusion criterion or an exclusion criterion.

The method 100 further comprises, in operation 120, accessing a dataset comprising a plurality of data items for each of a plurality of patients. The plurality of data items may comprise at least one first data item corresponding to the at least one trial outcome, and at least one second data item corresponding to the set of patient selection criteria. For example, the at least one second data item may correspond to the at least one primary criterion. In other words, the dataset includes data item(s) which may be used to assess the at least one trial outcome, and data item(s) which may be used to identify patients of the dataset according to patient selection criteria. In some instances, there may be a one-to-one mapping between trial outcome and first data item. For example, a trial outcome may be assessable by considering a single data item. In other instances, a single trial outcome may be associated with multiple first data items. The term 'first data item' is used throughout this disclosure to refer to data items, e.g. data elements, present in the dataset which may be used to assess a trial outcome. Similarly, the at least one second data item may be associated with a respective at least one criterion of the set of patient selection criteria (e.g. the at least one primary criterion), or a criterion of the set of patient selection criteria may correspond to multiple second data items. For example, a criterion may not be directly associated with a single data element, and a combination of data elements (e.g. data items) may be needed to assess whether a criterion is met.

In some embodiments, the method may comprise obtaining a mapping between the at least one trial outcome and one or more first data items of the dataset. Such a mapping may be obtained, for example, as a user input, from an internal memory, from an external source such as an external server or network. For example, a trial outcome relating to a patient's quality of life may be mapped to a data item representing hospital admissions within 5 years of treatment. In some embodiments, the method may comprise identifying at least one quantitative indicator for the at least one trial outcome and identifying at least one first data item of the plurality of data items based on the identified at least one quantitative indicator. For example, a trial outcome may not be directly quantitatively assessed. The trial outcome may instead be assessed by one or more quantitative indicators.

Similarly, in some embodiments, the method may comprise obtaining a mapping between one or more of the criteria of the set of patient selection criteria and one or more second data items of the dataset.

The method 100 further comprises, in operation 130, identifying a set of patients from the plurality of patients in the dataset based on the at least one second data item. That is, patients are selected from the plurality of patients in the dataset if their data items corresponding to the patient selection criteria meet the criteria.

In some embodiments, the dataset comprises real world data. Real world data may comprise a large number of data types (e.g. in some cases, more than 1000 data types). The data may be anonymized. In some embodiments, if one or more data items are missing from the dataset, a prediction algorithm may be used to predict the missing one or more data items.

The method 100 further comprises, in operation 140, performing a clustering analysis on the set of patients to identify a cluster of patients. In some instances, multiple clusters of patients may be identified. In such instances, the subsequent operations of the method (operations 150 to 190) may be performed for each of the identified clusters, as will be described below.

In other words, operation 140 comprises identifying one or more clusters of patients by performing a clustering analysis.

The clustering analysis may be performed using any suitable algorithm or method. The clustering analysis may be performed using a machine learning cluster analysis, such as simple k-means. Additional cluster algorithms include supervised algorithms, such as random survival forest, and/or unsupervised algorithms such as deep embedded clustering, or a mixture - for example, semi-supervised clustering (such as deep semi-supervised embedded clustering). Supervised algorithms may focus on one or more defined trial outcomes, whereas unsupervised algorithms may focus on identifying similarities between patient items. Further examples of cluster algorithms include density-based cluster algorithms (DBSCAN) and hierarchical-based cluster algorithms. It is to be understood that this list of cluster algorithms is not exhaustive, and any suitable cluster algorithm may be used herein.

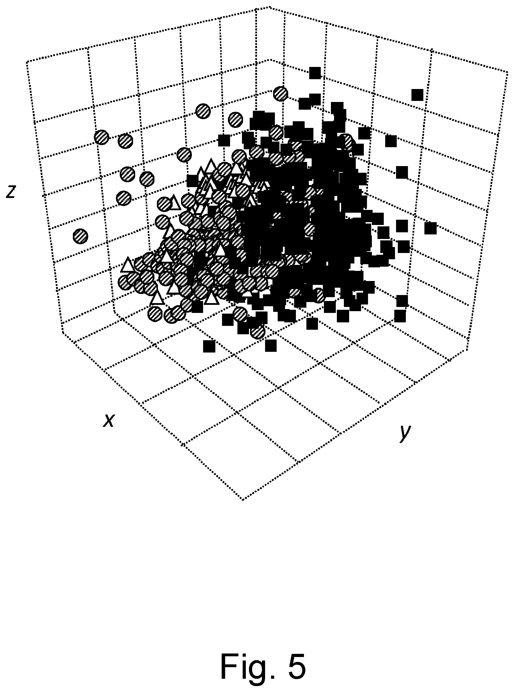

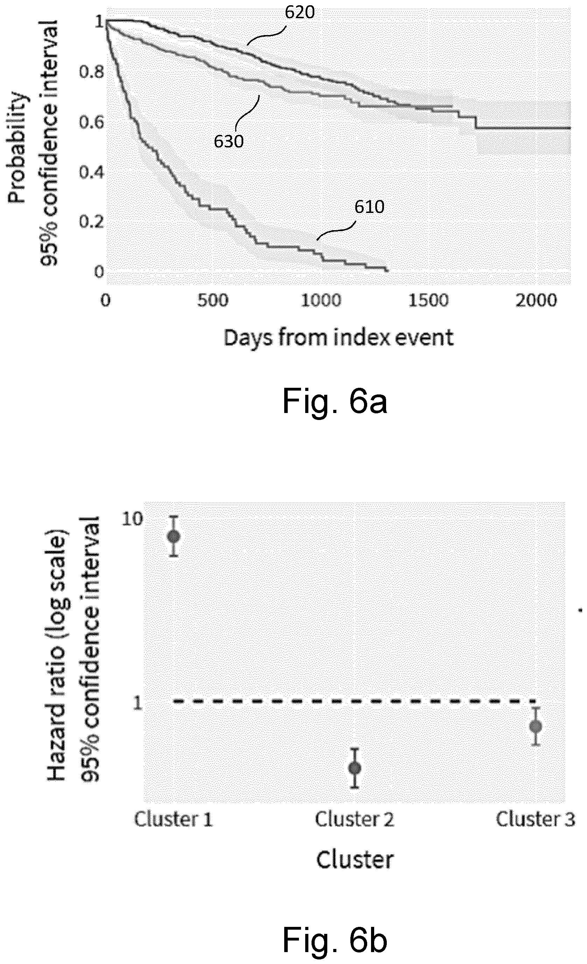

The method 100 further comprises, in operation 150, determining whether the identified cluster is clinically meaningful. The clinical meaningfulness may be assessed using a range of approaches. The identified cluster may be statistically evaluated with respect to other patient group(s), such as the full plurality of patients of the dataset, and/or any other identified clusters. For example, determining whether a cluster is clinically meaningful may comprise calculating a statistical significance score and comparing the statistical significance score to a threshold statistical significance. As another example, the determination of clinical meaningfulness may comprise performing a feature enrichment analysis. Further examples of statistical evaluation methods include enrichment analysis such as the Mann Whitney U test, and time-to-event analysis, such as a log rank test. In some embodiments, clinical meaningfulness may be determined by statistically evaluating the identified cluster with respect to at least one: the set of patients (e.g. the set of patients identified in operation 130 or 230), the other clusters identified in the clustering analysis, and/or one or more clinical trial outcomes. One example of the evaluation of clinical meaningfulness will be described in greater detail with reference to Figures 5, 6a and 6b.

In some embodiments, operation 150 may comprise obtaining user confirmation of clinical meaningfulness.

In some embodiments, operation 150 may comprise outputting an indication of clinical meaningfulness, such as a statistical score, to a user, and receiving a user input confirming or indicating that the cluster is statistically significant.

A clinically meaningful cluster may identify patients for which the clinical trial outcome may be particularly significant or more readily measurable. This may highlight further selection criteria which can improve patient recruitment in a clinical trial, in order to improve the demonstrability of a clinical trial outcome.

If it is determined that the cluster is clinically meaningful, the method 100 further comprises, in operation 160, determining at least one additional patient selection criterion based on the cluster. The cluster may identify patients which have similar values (e.g. values within a range) in a further data item (or further data items) (i.e. other than the second data items) of the plurality of data items. The further data item(s) correspond to one or more additional patient selection criteria, which may be used to select patients.

The method 100 further comprises, in operation 170, modifying the set of patient selection criteria by adding the one or more additional patient selection criteria. That is, the newly identified additional patient selection criteria are added to the set of patient selection criteria.

In some implementations, the method 100 may further comprise, in operation 180, outputting the modified set of patient selection criteria to a user. Outputting the modified set of patient selection criteria may comprise displaying the modified set of patient selection criteria on a screen, sending a report indicating the modified set of patient selection criteria to a user, or the like. In some implementations, the modified set of patient selection criteria may be additionally or alternatively output to another system, or to another component of the computing environment.

In some implementations, the method 100 may further comprise, in operation 190, selecting patients according to the modified set of patient selection criteria. Patients may be selected from the dataset, and/or from additional patient databases. For example, the modified set of patient selection criteria may be used to select patients within a geographic region using a centralized healthcare database, in order to make the clinical trial logistically more feasible. For example, the method 100 may further comprise designing a clinical trial using the modified set of patient selection criteria.

In some implementations, both operations 180 and 190 may be included. In other implementations, one of these operations may be included. For example, operation 190 of selecting patients may be performed regardless of whether the operation 180 is performed. That is, patients may be selected automatically (for example, from linked databases or datasets), without needing to output the modified set of patient selection criteria to the user. It is to be noted that both operations 180 and 190 are optional, as illustrated by the dashed lines in Figure 1. If both operations are performed, they may be performed in any order, simultaneously or substantially simultaneously.

**Figure 2** illustrates a flowchart of a method of determining patient selection criteria for a clinical trial according to embodiments of the disclosure. The operations of Figure 2 may be combined with the operations of Figure 1.

Referring now to Figure 2, additional optional operations of method 100 are described. As indicated in the description of Figure 1 above, the set of patient selection criteria comprises at least one primary criterion, and may further comprise at least one secondary criterion. For example, the at least one primary criterion may be referred to as core criteria - these are criteria which may be considered essential for a particular clinical trial. For example, if the clinical trial relates to a treatment for diabetes, one core criteria may be that the patients selected must have a diagnosis of diabetes. The at least one secondary criterion may be considered non-core criteria. In this example, a secondary criterion may be that the patients selected should be at least 50 years of age. The distinction of whether a criterion is a primary criterion or a secondary criterion (e.g. core or non-core) may be determined based on a user input, a flag or parameter in a database from which patient selection criteria are obtained, or the like.

The method 100 may further comprise, in operation 212, identifying one or more secondary criteria in the set of patient selection criteria. This operation may be performed during or after operation 110 (obtaining patient selection criteria), or before or during operation 130 (identifying the set of patients). The one or more secondary criteria may correspond to one or more further data items in the dataset. For example, each of the secondary criteria may be associated with, or mapped to, one or more further data items in the dataset.

The method 100 may further comprise, in operation 214, removing at least one secondary criterion from the set of patient selection criteria. In some implementations, all secondary criteria may be removed from the set of patient selection criteria. In other implementations, one or more secondary criteria may be removed from the set of patient selection criteria. In some implementations, the determination of which secondary criterion (or criteria) to remove may be based on a user input, a ranking, an importance or priority level, or the like.

The method 100 may further comprise, in operation 230, identifying a set of patients using the at least one primary criterion and any remaining secondary criteria (if present) in the set of patient selection criteria. Operation 230 may be performed as part of operation 130 of Figure 1. That is, the set of patients from the database may be identified based on the values of the at least one second data item and the further data item(s) which correspond to the remaining secondary criteria.

The method 100 may further continue to operations 140 and 150 to identify a cluster (or multiple clusters) of patients by performing a cluster analysis and subsequently determining if the cluster(s) is clinically meaningful, as described with reference to Figure 1.

If the cluster is determined to be clinically meaningful, the method 100 may further comprise, in operation 260, determining one or more additional criteria based on the cluster, as described with reference to operation 160 in Figure 1. In some implementations, the one or more additional criteria may be the same as one or more of the secondary criteria which were removed in operation 214. In these cases, the secondary criteria matching the one or more additional criteria may be validated. In some implementations, the one or more additional criteria may be similar to one or more of the secondary criteria removed in operation 214. In such cases, the removed secondary criteria which are similar to the additional criteria may, in essence, be refined or adjusted accordingly. In some implementations, the one or more additional criteria may differ from the secondary criteria removed in operation 214.

The method may further comprise, in operation 270, modifying the set of patient selection criteria by removing the at least one secondary criterion (i.e. the at least one secondary criterion removed in operation 214) and adding the one or more additional criteria determined in operation 260.

Although not shown in Figure 2, in some implementations, the method may optionally continue to one or more of operations 180 and 190, as described with reference to Figure 1.

**Figure 3** illustrates an example dataset according to embodiments of the disclosure. The dataset may comprise entries for a plurality of patients 310. For each patient in the plurality of patients, the dataset may comprise a plurality of data items 320. In some cases, the dataset may be incomplete for one or more patients. That is, a patient's data in the dataset may be missing one or more values for data items. For example, some information may not be available or applicable.

The plurality of data items 320 comprises at least one first data item 330 which may be associated with one or more trial outcomes. For example, the one or more first data item 330 may be used to assess whether the one or more trial outcomes is achieved, during or after completion of, a clinical trial. The plurality of data items 320 further comprises at least one second data item 340 which may be associated with one or more patient selection criteria, e.g. at least one primary criterion. In some embodiments, the plurality of data items 320 may further comprise at least one further data item (not shown) associated with one or more secondary criteria. The at least one second data item 340 and, if applicable, the at least one further data item, may be used to identify and select patients for recruiting to the clinical trial. That is, the set of patient selection criteria may initially comprise the primary criterion (or criteria) 340, and optionally, the secondary criteria if present.

In some embodiments, one or more data items of the plurality of data items may be used as both a first data item and a second data item. That is, in some embodiments, the same data item may be used in the selection of patients and in the assessment of the clinical trial outcome. For example, a clinical trial into a blood pressure medication may require patients having a blood pressure within a particular range, and the same clinical trial may be, at least in part, assessed by determining if blood pressure has, for example, decreased. In this example, the 'blood pressure' data item would be used both to select patients for the clinical trial and to determine if the clinical trial outcome was met. A further example of a criterion used to determine if a clinical trial outcome may be a date of death, even though a date of death is not always present for every patient.

Upon performing the clustering analysis (operation 140 in Figures 1 or 2) and determining that a cluster is clinically significant (operation 150 in Figures 1 or 2), an additional criterion is determined based on the identified cluster (operation 160 in Figure 1; operation 260 in Figure 2). The additional criterion may be determined by identifying a data item 350 (or multiple data items) of the plurality of data items in the dataset which may be used to define describe the cluster. For example, the cluster analysis may be used to identify a cluster of patients, the patients of which having a value for a data item falling within a particular range. In other words, the data item 350 may be used to identify patients of the cluster from the set of patients initially identified. Determining the additional criterion may comprise first determining the one or more data items of the dataset (in the example herein, this is indicated by reference sign 350), and then determining an additional criterion associated with the determined one or more data items (e.g. reference sign 350). The additional criterion may comprise, for example a range of values within which the one or more data items 350 should fall.

The additional criterion may then be added to the set of patient selection criteria, as described with reference to operation 170 in Figure 1 (or similarly, operation 270 of Figure 2). During patient selection for a clinical trial, patients may be selected (e.g. recruited) for the clinical trial based on, in this example, the values for data items 340 and 350. That is, patients may be subsequently selected for a clinical trial based on the modified set of patient selection criteria, which includes the primary criteria and the additional criterion (or criteria), and in some embodiments, one or more secondary criteria.

**Figure 4** illustrates a flowchart of further operations of a method of determining patient selection criteria for a clinical trial according to embodiments of the disclosure. The operations of method 400 may be combined with the operations of method 100 described with reference to Figures 1 and 2. In particular, Figure 4 describes an example of a method for determining one or more additional patient selection criteria (e.g. in operation 160 of Fig. 1 or operation 260 in Fig. 2) based on the cluster identified in operation 140 (in Figure 1 or 2). It is to be noted, however, that this method is merely exemplary and other methods may be used in addition to, or as an alternative to, the method described herein. In some embodiments, the use of method 400 may be used to explain the model. For example, the use of method 400 may be used, or omitted, based on the cluster method used. That is, if the cluster method uses, for example, a k-means clustering algorithm, the operations of method 400 may be omitted as the cluster method is simple and interpretable, and the use of a surrogate model as described below may not provide a substantial benefit. In cases where the cluster method is not intrinsically interpretable, such as deep embedded clustering, the use of a surrogate model as described below is particularly advantageous to provide interpretability.

The method 400 may comprise, in operation 410, training a surrogate model. The surrogate model may be trained to predict one or more cluster labels of relevance. The surrogate model may be a supervised machine learning model which uses clinical features, such as diagnoses, laboratory values, medications, and the like, as inputs, and cluster labels of a clustering algorithm used in the clustering analysis as ground truth. The inputs may be provided to the surrogate model from the dataset, or from a subset of the dataset, e.g. corresponding to data of the identified set of patients. The surrogate model may be a simple-to-interpret model, such as a single decision tree or the like. The surrogate model may be trained using any appropriate architecture.

The clinical features used as inputs to the surrogate model may be obtained from the dataset. For example, the surrogate model may take the first data items and/or the second data items as inputs. In some embodiments, the first and/or second data items may be preprocessed before being input into the surrogate model. In some embodiments, the data items used in the clustering algorithm may be used as inputs to the surrogate model. In some embodiments, the data items used in the clustering algorithm may be preprocessed before being input into the surrogate model. For example, a clustering algorithm may use individual laboratory values as inputs, whereas the surrogate model may use a mean and standard deviation of the laboratory values as inputs. That is, the individual laboratory values (for example) may be used to determine the mean and standard deviation to be input into the surrogate model.

Operation 410 may be performed once clusters have been identified, for example after operation 160 in Figure 1 (or operation 260 in Figure 2).

The method 400 may further comprise, in operation 420, generating a set of inclusion and/or exclusion criteria. The set of inclusion and/or exclusion criteria may be based on the trained surrogate model. For example, the surrogate model may be a single decision tree. The one or more paths from a decision tree root to a leaf node which has a majority class containing the cluster may be used. In other words, at each decision point along the tree, a feature (e.g. a data item) is used to split the set of patients. That is, at each decision point in the tree, it is determined whether each patient has the feature corresponding to that decision point (e.g. whether, for each patient, the value for the data item is within a particular range, has a particular value, or the like). Patients whose data does not have the feature corresponding to the decision point may be excluded or discarded - in other words, they are not considered at subsequent decision points. Patients whose data does have the feature corresponding to the decision point are maintained, and are then considered at the next decision point corresponding to a further feature.

A set of decision paths may thus be generated for every leaf node which has a majority class containing the cluster identified in operation 140. The set of decision paths may comprise at least one decision path. The generated set of decision paths may be used to provide a set of inclusion and/or exclusion criteria. At this stage, the set of inclusion and exclusion criteria may be considered an initial set of inclusion and/or exclusion criteria.

The method 400 may further comprise, in operation 430, modifying the set of inclusion and/or exclusion criteria. This may comprise providing an interactive interface to a user, to enable the user to modify the set of inclusion and/or exclusion criteria. For example, the interactive interface may provide the set of inclusion and/or exclusion criteria to a user. That is, initially, the initial set of inclusion and/or exclusion criteria may be provided to the user. Criteria may then be added and/or removed from the set of inclusion and/or exclusion criteria. The interactive interface may adaptively display an indication of clinical meaningfulness of the set of inclusion and/or exclusion criteria to the user. For example, the interactive interface may display an indication of clinical meaningfulness based on the inclusion and/or exclusion criteria included in the set. If the user adds or removes a criterion from the set of inclusion and/or exclusion criteria, the indication of clinical meaningfulness may be updated to reflect the change in the set of inclusion and/or exclusion criteria. The indication of clinical meaningfulness may be determined, for example using any of the methods described herein, such as those described with reference to Figures 5, 6a and 6b. The user may choose to adjust the set of inclusion and/or exclusion criteria to remove clinically irrelevant features, decrease the complexity, and/or adjust the cohort outcome, for example. The interactive interface may enable the user to evaluate the impact of the selected inclusion and/or exclusion criteria based on, for example, a time to event analysis - e.g. survival compared to the original model and surrogate model cluster labels.

In some embodiments, the method 400 may comprise providing an explainability output. For example, the method 400 may output a decision tree and/or confusion matrix which may be used to explain the surrogate model, which may improve the understandability, and therefore the interpretation, of the model.

In some embodiments, the user may confirm the set of inclusion and/or exclusion criteria, for example after having added and/or removed one or more criteria to the set.

In some embodiments, the set of inclusion and/or exclusion criteria may be confirmed using, for example, an artificial intelligence system or method may be used to determine whether to a feature is to be included in the set of inclusion and/or exclusion criteria, for example based on the indication of clinical meaningfulness.

Once it has been determined which criteria to include and/or exclude in the set of inclusion and/or exclusion criteria, the set of inclusion and/or exclusion criteria may be provided as the additional criteria, to be included in the modified set of patient selection criteria (in operation 170 of Figure 1; operation 270 of Figure 2).

**Figure 5** illustrates an example cluster visualization according to embodiments of the disclosure. That is, the Figure 5 illustrates the visualization of a cluster using one example of a cluster algorithm, used in operation 140 of Figures 1 and 2. In this example, a machine learning based cluster algorithm is described to find one or more clusters of patients. Performing the clustering analysis may, in some instances, comprise performing one or more pre-processing steps and/or selecting a cluster algorithm, for example based on the clinical trial and/or type(s) of data used. That is, performing the clustering analysis may comprise selecting a clustering algorithm based on one or more of: data types (e.g. diagnoses codes or diagnoses, procedures, and/or medications, etc.), the part of the patient trajectory to consider (e.g. data around a specific event, data filtered from a set time period such as within the last 30 days, or data recorded before a specific event), and/or how data is pre-processed (e.g. how data is filtered, how missing data is handled, how features are generated). The patient trajectory may also be referred to as a patient history, and may comprise a record of clinical events, such as procedures, medications taken or prescribed, diagnoses and the like, provided with a timestamp. In some embodiments, the clustering algorithm to be used may be selected by receiving a user input indicating a particular clustering algorithm, or may be selected automatically.

Performing the clustering analysis may comprise outputting a patient-to-cluster-ID table, which maps each patient of the set of patients identified in operation 130 (Figure 1) or operation 230 (Figure 2) to a specific cluster. Figure 5 illustrates a three-dimensional visualization showing three clusters, distinguished as white-filled triangles (cluster 1), hashed circles (cluster 2) and solid black squares (cluster 3).

Once one or more clusters has been identified in operation 140 (Figures 1 and 2), the clinical meaningfulness of the one or more clusters may be evaluated. That is, the cluster(s) are analyzed to determine if they are clinically relevant. Determining if a cluster is clinically relevant is particularly advantageous when the dataset comprises real world data, as real world data is typically noisy and can, in some cases, incorporate data biases. Such biases may be unknown. In some embodiments, synthetic data may be used, for example synthetic data generated from real world data.

The evaluation of the clinical meaningfulness of the cluster may comprise performing a time-to-event analysis, for example using Kaplan Meier curves. In some embodiments, Cox hazard models may be used.

**Figures 6a** and **6b** illustrate example plotted analyses of patient clusters according to embodiments of the disclosure. In particular, Figure 6a illustrates Kaplan Meier curves for a survival analysis of the patient clusters illustrated in Figure 5, and Figure 6b illustrates Cox model hazard ratio estimates (1-v-Rest) for a survival analysis of the patient clusters illustrated in Figure 5.

Referring first to Figure 6a, cluster 1, indicated by reference 610, can be seen to be enriched with the event of interest in this particular example. In the illustrated example, the curves illustrate the survival of patients as time progresses from a particular event. Time is indicated in days from a particular event (the index event) along the x-axis, and the probability of survival (in this example) is indicated on the y-axis. For cluster 1 (indicated by reference numeral 610), the probability of survival can be seen to decrease significantly, especially compared to clusters 2 and 3 (reference numerals 620 and 630, respectively). This type of distinction of the probability of survival between cluster 1 (reference numeral 610) and the other clusters 620 and 630 may indicate that there is a particular feature, or data item, shared by the patients in cluster 1 (reference numeral 610). A clinical trial performed using a patient selection criteria corresponding to cluster 1 (reference numeral 610) may be particularly well suited as a control for the treatment with a particular drug for improving survival after a particular event. As cluster 1 (reference numeral 610) has a worse survival outcome compared to the other two clusters in this example, benefits of the treatment being tested may be more readily observable. As a consequence, less patients would need to be recruited in order to statistically demonstrate the benefit of the treatment. That is, since the probability of survival decreases rapidly, any improvement in survival observed in the clinical trial may be more statistically observable (e.g. the clinical significance of such an improvement may be clearer) than if the clinical trial were performed, for example, using the patient selection criteria defining the patient set as a whole (e.g. including clusters 1, 2 and 3 in this example), or using patient selection criteria corresponding to clusters 2 and/or 3. Additionally, the duration of the trial may be reduced. In some clinical trials, for example, a predetermined number of outcome events may be required, which may be longer if clusters 2 and/or 3 are used in comparison to cluster 1.

Figure 6b illustrates Cox model hazard ratio estimates for the clusters illustrated in Figure 5. Clusters 1, 2 and 3 are indicated in Figure 6b using the same reference signs as in Figure 6a, although it is to be appreciated that these analyses need not both be performed.

The Cox model provides an estimate of the hazard ratio, and its confidence interval, which illustrates the ratio of hazard rate in a particular cluster versus the rest of the clusters. As in Figure 6a, cluster 1 in Figure 6b shows a significantly higher hazard ratio compared to the other clusters. The use of the Cox model may also include additional factors such as sex and age in order to ensure that the result of the model is statistically significant. This may provide a way to clinically evaluate the results.

The clinical meaningfulness of a cluster identified in operation 140 may be evaluated using various approaches. For example, a feature enrichment may be used to identify features which are either over- or under-represented in the identified cluster, compared to the set of patients (e.g. patients across all clusters, or the set of patients identified in operations 130 or 230). In some embodiments, a feature enrichment table may be output to a user, for example using the interactive interface described with reference to Figure 4. The feature enrichment table may provide a cluster breakdown, and may indicate one or both of highly enriched features and under-enriched features for each cluster.

In some embodiments, the method (e.g. method 100) may further comprise comparing the modified set of patient selection criteria to the initial set of patient selection criteria (e.g. the set of patient selection criteria prior to modification, or the set of patient selection criteria obtained in operation 110). Evaluating the modified set of patient selection criteria may be used to check whether the criteria are too severe, which could result in too few patients being available to be recruited, whether the criteria are too relaxed, which could result in a decrease in the statistical significance of the trial.

Comparing the modified set of patient selection criteria to the initial set of patient selection criteria may be used to evaluate the impact of the modifications of the patient selection criteria on one or more key clinical trial performance indicators, such as the number of patients required for the clinical trial (e.g. cohort size), trial enrolment rate, or estimated trial duration.

The comparison may comprise using a power analysis to compare a number of patients required using the modified set of patient selection criteria to the number of patients required using the initial set of patient selection criteria (i.e. the set of patient selection criteria obtained in operation 110). Using the modified set of patient selection criteria may result in a lower number of patients required. For example, the modified set of patient selection criteria may be used to identify a subset of the patients which would be identified using the initial set of patient selection criteria.

The comparison may additionally or alternatively comprise determining a trial enrolment rate, e.g. the number of patients to be recruited per month, using the modified set of patient selection criteria, and using the initial set of patient selection criteria. The trial enrolment rate determined using the modified set of patient selection criteria may be compared to the trial enrolment rate determined using the initial set of patient selection criteria, to determine the impact of the modified set of patient selection criteria on trial enrolment rate.

The comparison may additionally or alternatively comprise determining an estimated trial duration using the modified set of patient selection criteria and using the initial set of patient selection criteria. For example, a clinical trial designed using the modified set of patient selection criteria may have a shorter duration than a similar clinical trial designed using the initial set of patient selection criteria.

In some embodiments, if multiple clusters are identified in operation 140, the evaluation may be performed for each cluster. This evaluation may be automated, and the most relevant clusters may be identified based on the result of the evaluation.

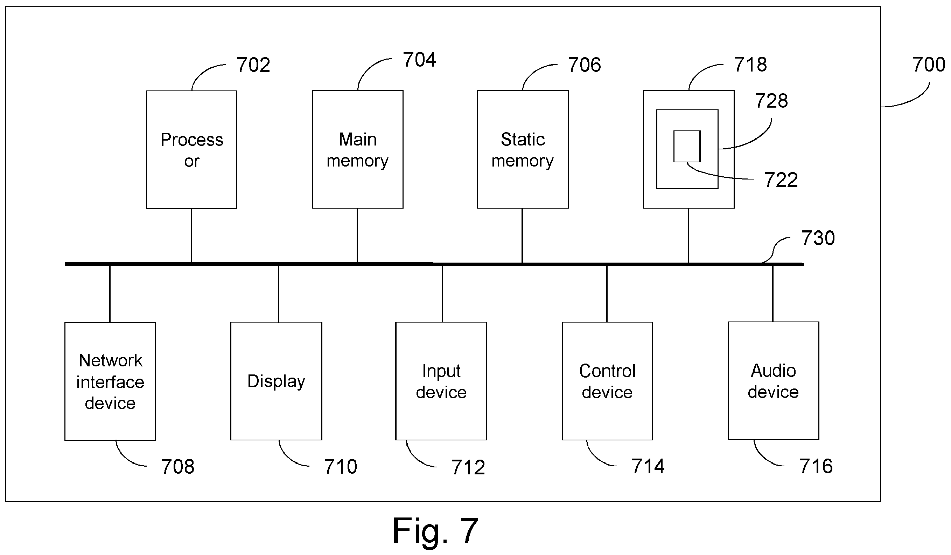

**Figure 7** illustrates a block diagram of one implementation of a computing device 700 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 700 includes a processing device 702, a main memory 704 (e.g., read-only memory (ROM), flash memory, dynamic random access memory (DRAM) such as synchronous DRAM (SDRAM) or Rambus DRAM (RDRAM), etc.), a static memory 706 (e.g., flash memory, static random access memory (SRAM), etc.), and a secondary memory (e.g., a data storage device 718), which communicate with each other via a bus 730.

Processing device 702 represents one or more general-purpose processors such as a microprocessor, central processing unit, or the like. More particularly, the processing device 702 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 702 may also be one or more special-purpose processing devices such as an application specific integrated circuit (ASIC), a field programmable gate array (FPGA), a digital signal processor (DSP), network processor, or the like. Processing device 702 is configured to execute the processing logic (instructions 722) for performing the operations and steps discussed herein.

The computing device 700 may further include a network interface device 708. The computing device 700 also may include a video display unit 710 (e.g., a liquid crystal display (LCD) or a cathode ray tube (CRT)), an alphanumeric input device 712 (e.g., a keyboard or touchscreen), a cursor control device 714 (e.g., a mouse or touchscreen), and an audio device 716 (e.g., a speaker).

The data storage device 718 may include one or more machine-readable storage media (or more specifically one or more non-transitory computer-readable storage media) 728 on which is stored one or more sets of instructions 722 embodying any one or more of the methodologies or functions described herein. The instructions 722 may also reside, completely or at least partially, within the main memory 704 and/or within the processing device 702 during execution thereof by the computer system 700, the main memory 704 and the processing device 702 also constituting computer-readable storage media.

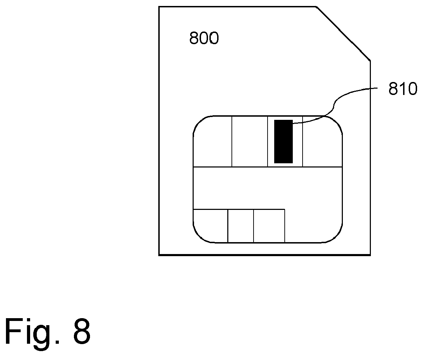

The various methods described above may be implemented by a computer program. The computer program may include computer code arranged to instruct a computer to perform the functions of one or more of the various methods described above. The computer program and/or the code 810 for performing such methods may be provided to an apparatus, such as a computer, on one or more computer readable media or, more generally, a computer program product, depicted in **Figure 8****.** The computer readable media 800 may be transitory or non-transitory. The one or more computer readable media 800 could be, for example, an electronic, magnetic, optical, electromagnetic, infrared, or semiconductor system, or a propagation medium for data transmission, for example for downloading the code over the Internet. Alternatively, the one or more computer readable media 800 could take the form of one or more physical computer readable media such as semiconductor or solid state memory, magnetic tape, a removable computer diskette, a flash drive, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "assigning", "finding," "imputing", "identifying" or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A computer-implemented method for determining patient selection criteria for a clinical trial, the clinical trial having at least one trial outcome, the method comprising:
obtaining a set of patient selection criteria, the set of patient selection criteria comprising at least one primary criterion;
accessing a dataset comprising a plurality of data items for each of a plurality of patients, the plurality of data items comprising at least one first data item corresponding to the at least one trial outcome and at least one second data item corresponding to the set of patient selection criteria;
identifying a set of patients of the plurality of patients in the dataset based on the at least one second data item;
performing a clustering analysis on the set of patients to identify a cluster of patients;
determining whether the identified cluster of patients is clinically meaningful; and
upon determining that the identified cluster of patients is clinically meaningful:
determining at least one additional patient selection criterion based on the identified cluster of patients, and
modifying the set of patient selection criteria by adding the at least one additional patient selection criterion to the set of patient selection criteria.

2. The method of claim 1, further comprising outputting the modified set of patient selection criteria to a user.

3. The method of claim 1 or claim 2, further comprising selecting patients according to the modified set of patient selection criteria.

4. The method of any preceding claim, wherein performing the cluster analysis comprises using at least one of a machine learning cluster analysis, such as simple k-means.

5. The method of any preceding claim, wherein determining at least one additional patient selection criterion further comprises:
training a surrogate model using the plurality of data items;
generating a set of inclusion and/or exclusion criteria based on the trained surrogate model;
modifying the set of inclusion and/or exclusion criteria by generating and outputting an interactive user interface enabling features to be added and/or subtracted from the set of inclusion and/or exclusion criteria and adaptively displaying an indication of clinical meaningfulness of the set of inclusion and/or exclusion criteria, and obtaining user input via the interactive user interface; and
providing the modified set of inclusion and/or exclusion criteria as the at least one additional patient selection criterion.

6. The method of any preceding claim, wherein determining whether the identified cluster of patients is clinically meaningful comprises calculating a statistical significance score and comparing the calculated statistical significance score to a threshold statistical significance.

7. The method of any preceding claim, wherein determining whether the identified cluster of patients is clinically meaningful comprises statistically evaluating the identified cluster with respect to at least one of the set of patients and/or any other identified clusters.

8. The method of any preceding claim, wherein determining whether the identified cluster of patients is clinically meaningful comprises performing a feature enrichment analysis and/or a time-to-event analysis.

9. The method of any preceding claim, wherein determining whether the identified cluster of patients is clinically meaningful comprises determining if one or more features are over- or under-represented in the cluster compared to the set of patients, the one or more features being associated with one or more data items of the plurality of data items.

10. The method of any preceding claim, wherein the set of patient selection criteria further comprises at least one secondary criterion, the at least one secondary criterion associated with a further at least one data item of the plurality of data items, wherein identifying the set of patients of the plurality of patients in the dataset comprises:
removing one or more of the at least one secondary criterion from the set of patient selection criteria; and
identifying the set of patients of the plurality of patients in the dataset based on the second data item and the further at least one data items associated with any remaining secondary criteria; and
wherein modifying the set of patient criteria further comprises removing the one or more of the at least one secondary criterion from the set of patient selection criteria.

11. The method of any preceding claim, further comprising at least one of:
obtaining a first mapping between the set of patient selection criteria and the plurality of data items and identifying the at least one second data item corresponding to the set of patient selection criteria according to the obtained first mapping; and/or
obtaining a second mapping correlating the at least one trial outcome and at least one data item of the plurality of data items and identifying the at least one first data item according to the obtained second mapping.

12. The method of any preceding claim, further comprising identifying at least one quantitative indicator for the at least one trial outcome and identifying at least one first data item of the plurality of data items based on the identified at least one quantitative indicator.

13. The method of any preceding claim, wherein performing the cluster analysis comprises selecting a cluster analysis algorithm based on at least one data type of the at least one first data item and/or the at least one second data item.

14. A transitory or non-transitory computer-readable medium comprising instructions which, when executed by a computer, cause the computer to carry out the method of any one of the preceding claims.

15. A device comprising:
an input/output subsystem configured to transmit and receive data;
one or more processors;
a memory comprising instructions which, when executed, cause the one or more processors to execute the method of any one of claims 1 to 13.
